# EUROPEAN PATENT SPECIFICATION

(11) **EP 0 512 191 B1**
(45) Date of publication and mention of the grant of the patent: **01.04.1998**
(21) Application number: 92100055.0
(22) Date of filing: 03.01.1992
(51) Int. Cl.: A61B 17/06

(54) **Package for surgical sutures**
Verpackung für chirurgisches Nahtmaterial
Emballage pour des sutures chirurgicales

(30) Priority: 03.01.1991 US 637186; 09.05.1991 US 697757
(43) Date of publication of application: 11.11.1992
(73) Proprietor: United States Surgical Corporation, Norwalk, Connecticut 06856 (US)
(72) Inventor: Holzwarth, Henry A., Weston, CT 06883 (US); Scanlon, Christopher M., Milford, CT 06460 (US)
(74) Representative: Marsh, Roy David

(56) References cited:
- EP-A- 0 055 823
- WO-A-89/07420
- DE-A- 3 735 649
- US-A- 2 583 043
- US-A- 4 135 623

## Description

### BACKGROUND OF THE INVENTION

### 1. Field of the Invention

The present invention relates to packaging devices for surgical elements such as sutures and suture-needle assemblies, and more particularly to packages for monofilament-type sutures which are to be packaged in an unfolded and elongated condition. A package in accordace with the pre-characterising part of claim 1 is disclosed in US-A-4135623.

### 2. Discussion of the Prior Art

Packages for surgical sutures and suture-needle assemblies having means for retaining the suture in the package are well known in the art. These packages typically provide a series of panels which are foldable about each other to enclose the suture within. In most cases, means are provided on the package for securing the needle in place, positioning the suture within the package where the suture is restrained from moving once the package is closed.

In general, most suture packages in the prior art provide a retainer member consisting of a series of panels having perforated score lines which allow one panel to fold over another panel to close the retainer with the suture and needle packaged therein. Typically, the suture is wound in an oval or figure "8" pattern which reduces the size of the package and provides an efficient means for storing the sutures prior to use. Other packages provide a series of elongated loops which allows the suture to be stored in a relatively flattened condition with only a few bends in the suture material.

In the surgical suture and suture-needle industry, a significant amount of emphasis has recently been placed on the condition of the suture when it is removed from the package. The "memory" retention of the suture has become an important feature in the practical use of sutures in an operating room, in that emphasis has been placed on the necessity of sutures having little or no bends or creases after they are removed from the package. In general, sutures that are wound in small packages in a figure "8" or oval pattern will exhibit numerous bends and creases along their length after they are removed from the package. This leads to an awkward suturing and stitching process in the operating room, in that the operating room personnel must first straighten the suture material to assist the surgeon in the process. Furthermore, the bends and creases may lead to entanglement of the sutures, particularly in packages in which numerous sutures are enclosed.

Several types of sutures are prone to bending and creasing and as a result require packaging in individual paths or tracks within the suture package, which often leads to an expensive and elaborate package requiring additional assembly steps during manufacture. In particular, sutures constructed of plastic material such as polypropylene or sutures constructed of stainless steel easily develop creases during packaging which reduce the effectiveness of the suture in an operating room. Accordingly, it is important that these types of sutures be packaged with a minimal amount of bends or turns, in order to allow the suture to maintain its natural shape after removal from the package.

While some packages in the prior art provide for the suture to be packaged in an elongated condition, these packages require at least one turn or bend in the suture to secure the suture within the package, particularly in cases where the suture is a "double-armed" suture, having needles at both ends of the suture. The bend in suture packaged in this manner is usually a sharp, 180° turn, which usually remains in the suture after the suture is removed from the package.

The package for surgical sutures and suture-needle assemblies of the present invention provides an elongate package for accommodating suture material without folds and creases, the package having novel needle display means to make available an elongate suture which maintains its natural shape after the suture has been removed from the package.

### SUMMARY OF THE INVENTION

According to one aspect of the present invention, there is provided a suture package as defined in claim 1, below.

According to a second aspect of the present invention, there is provided a method of loading sutures into a suture package, as defined in claim 18, below.

In accordance with a third aspect of the present invention, claim 24 below defines a method for packaging suture-needle assemblies.

Claim 27, below defines a fourth aspect of the invention as a method of removing a suture-needle assembly from a package.

The present invention provides an elongate package for surgical sutures and suture-needle assemblies which allows the suture to maintain its natural shape during packaging as well as after the suture is removed from the package for use in an operating room. The package is particularly suited for use with sutures that easily crease upon bending, such as sutures constructed of plastic material (for instance, polypropylene) or stainless steel.
The package of the present invention which can be die-cut essentially comprises a sheet of packaging material such as paperboard, fiberboard, or any fibrous material such as Tyvek (a registered trademark of DuPont), which exhibits a series of interconnected panels. Preferably, the package of the present invention comprises three panels which are connected through scored or perforated lines which facilitate folding of the material about itself to form a package. A first of these three panels is provided with means for securing the suture and suture-needle assembly in an elongate and unfolded condition. The means for securing the sutures may be an elongate tubular member, preferably constructed of plastic or a rolled paperboard material, which provides a tubular housing for the sutures which essentially eliminates the possibility of creasing of the suture material. Such a tube is disclosed in DE-A-3735649. The tubular material can be secured to the first panel of the package in a series of overlapping die cuts in the material, which allows the material to grasp the tubular member to hold it in place. Alternately, an adhesive material or glue may be used to secure the tube to the first panel. The first panel may also be provided with a needle-retaining member, preferably a foam, adhesive-backed needle park which holds the needles in place within the package.

The second panel, which forms the front panel of the package, may be provided with a bottom flap which folds over to form a bottom of the package. At the other end of the second panel is a needle display means adjacent the needles, which allows the needles to be covered within the package when the package is closed, and facilitates display and removal of the needles from the package without having to fully open the package to remove the sutures and needles.

The third panel forms the back panel of the package and may be provided with a top extension flap which folds over the top of the package to completely enclose the needles. The third panel may also be provided with means for holding the package in a closed position, where the third panel cooperates with the first panel to maintain the package in a folded, closed condition. Locking means may be provided and generally comprises a tab and slot arrangement, but may also include adhesives such as heat activated adhesives to seal the third panel to the first panel to close the package. The tab may extend outwardly from the longitudinal edge of the third panel, but preferably is an inwardly die-cut tab, so that the longitudinal edge of the third panel is straight.

### BRIEF DESCRIPTION OF THE DRAWINGS

The foregoing features of the present invention will become more readily apparent and may be understood by referring to the following detailed description of an illustrative embodiment of the package for surgical sutures and suture-needle assemblies and its novel construction, taken in conjunction with the accompanying drawings, in which:
Fig. 1 is a top view of a surgical suture and suture-needle assembly package of the present invention in the unfolded condition;
Fig. 2 illustrates the surgical suture and suture-needle assembly package of Fig. 1 having suture-needle assemblies positioned in the package, still with the package in the unfolded condition;
Fig. 3 is a top view of part of the surgical suture and suture-needle assembly package of Fig. 2 in the folded condition showing the needles exposed;
Fig.4 is the top view of Fig. 3 but with the closure flap (26) in the folded condition;
Fig.5 is a top view of an alternate embodiment of the package having suture-needle assemblies positioned in the package with the package in the fully unfolded condition;
Fig. 6 is a top view of part of the package of Fig. 5 in the folded condition with the needles exposed; and
Fig. 7 is a top view of another embodiment of the package of the present invention in the unfolded condition; and
Fig. 8 is a top view of yet another embodiment of the package of the present invention in the unfolded condition.

### DETAILED DESCRIPTION OF THE PREFERRED EMBODIMENTS

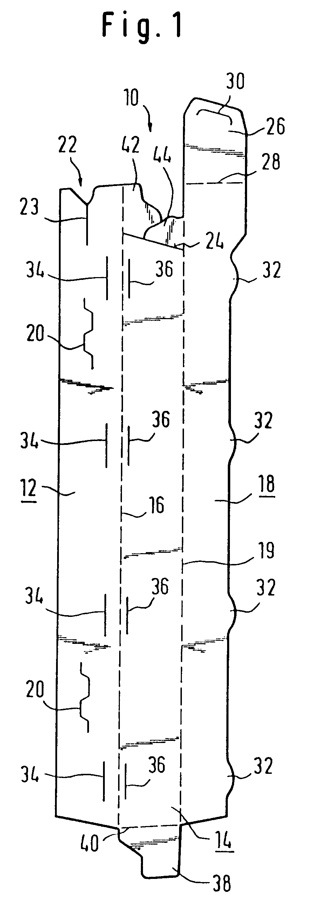

Referring now in specific detail to the drawings, in which like reference numerals identify similar or identical elements throughout the several views, Fig. 1 shows the package 10 of the present invention in the fully unfolded and unarmed condition. Package 10 essentially comprises a sheet of packaging material, preferably a fibrous material such as paper, paperboard, fiberboard, or Tyvek (a registered trademark of DuPont) which is a fibrous material constructed of spun bonded polyolefin fibers which are pressed together to form a sheet of fibrous material. Package 10 consists of a series of interconnected panels, and in the preferred embodiment comprises three panels which are foldable over each other through a series of scored or perforated fold lines.

A first panel 12 serves as a needle and suture retaining panel, and is connected to the second or center panel 14 through a score line 16. When the package is fully folded, center panel 14 forms the front of the package. Panel 14 is connected to third panel 18 through score line 19, and third panel 18 forms the back of the package when the package is in the fully folded condition.

Panel 12 is provided with a series of die cuts 20 which provide a means for retaining the surgical sutures to be packaged within package 10. Panel 12 is further provided with a cutout region 22 and a slit 23 at the top of the package whose function will be described later.

Second panel 14 is provided with an end closure flap 38 which extends from the body of the package and is foldable about the second panel 14 through score line 40. Flap 38 provides a bottom closure to the package. Second panel 14 is provided with an upper edge 24 which defines a needle display area.

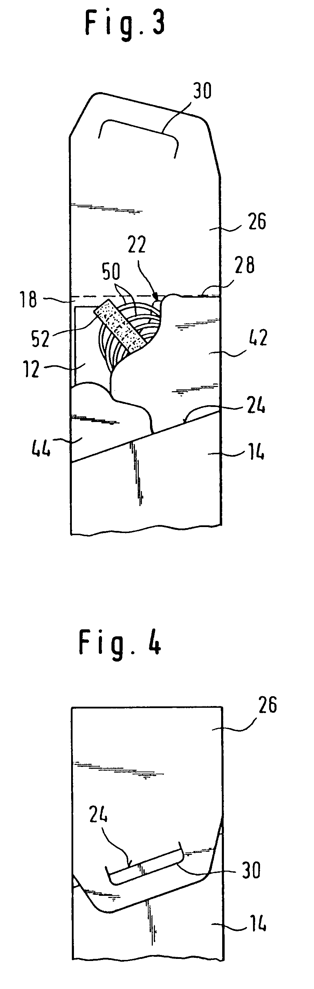

A pair of fold over flaps 42 and 44 provide a cover for the needle display area, and open along score lines 16 and 19 at edge 24. Flaps 42 and 44 are best seen in Fig. 3 wherein flap 42 covers the tips of the needles to further protect the needles within the package.

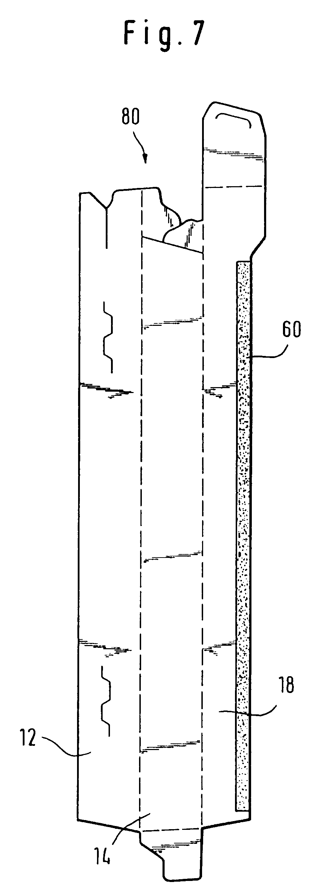

Third panel 18 is provided with a top closure flap 26 which is foldable about score line 28 to provide a cover for the needles when the package is fully folded. Locking tab 30 engages edge 24 of second panel 14 as best seen in Fig 4. Third panel 18 is provided with a series of tabs 32 for engaging slots 34 of first panel 12. The engagement of tabs 32 into slots 34 holds the package in the fully closed position. Tab 32 may be positioned to pass through slot 34 as well as slot 36 on second panel 14 to further secure the package. As an alternative to tabs 32 and slots 34 and 36, third panel 18 is provided with a strip of adhesive 60, as best seen in Fig. 7 , such as a heat or pressure sensitive adhesive material which will secure the third panel 18 to first panel 12 to hold the package in the fully closed position. In addition, slots 36 may be eliminated so that tabs 32 engage only slots 34 to hold the package in the closed position.

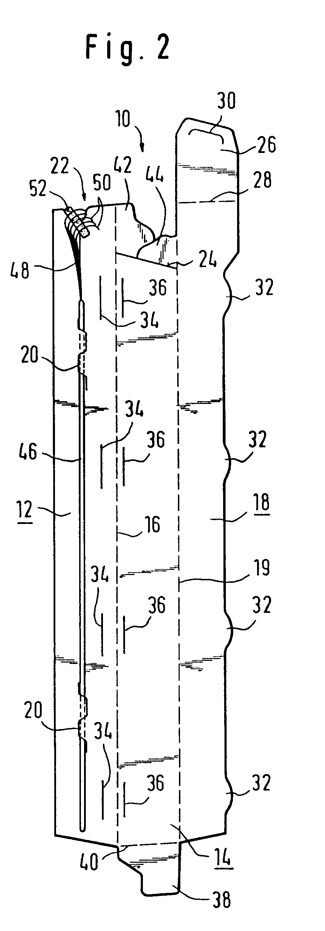

Fig. 2 shows package 10 of Fig. 1 having a number of suture-needle assemblies in position within the package with the package in the fully opened condition. In order to maintain the original shape of sutures 48, the sutures are packaged in a tubular member 46, which is preferably a plastic tube or a tube of rolled paper material which resists bending and folding. Tubular member 46 is held in place by die cuts 20 which provide a means for overlapping tubular member 46 to hold it in place. While any number of die cuts 20 may be provided, the preferred embodiment provides two cuts as seen in Fig. 2. It is also contemplated that die cuts 20 be eliminated and tubular member 46 be secured to first panel 12 through adhesives. In order to secure needles 50, a foam, adhesive-backed needle park 52 is provided which is positioned along an edge of cutout 22. Cutout 22 facilitates grasping of the needles when the package is in the fully folded condition.

Fig. 2 illustrates an alternate securement means for foam needle park 52 which secures needles 50 at cutout 22. In the embodiment shown in Fig. 2, needle park 52 overlaps cutout 22 so that as panel 12 is folded over panel 14, and panel 18 is folded over panel 12, the adhesive backed needle park 52 maybe secured directly to third panel 18 as best seen in Fig. 3.

Fig. 3 shows the package in the closed position with the needles 50 secured in foam needle park 52.

Needles 50 are displayed above edge 24 of second panel 14. As seen in Fig. 4, flap 26 is folded along score line 28 so that locking tab 30 engages edge 24 to close the package about the needles.

Tab 30 is positioned at an angle to scored line 28 which aligns tab 30 with edge 24 in parallel relation when flap 26 is folded over the needles.

Fig. 3 shows needles 50 secured in foam park 52 which overlaps cutout 22 of panel 12. In this manner, the adhesive-backed needle park is secured to both first panel 12 and third panel 18 as shown. Closure flaps 42 and 44 fold over to protect the needle tips and sutures as shown. To close the package, flap 26 is folded along score line 28 so that tab 30 engages edge 24 of second panel 14 in the manner as illustrated in Fig. 4.

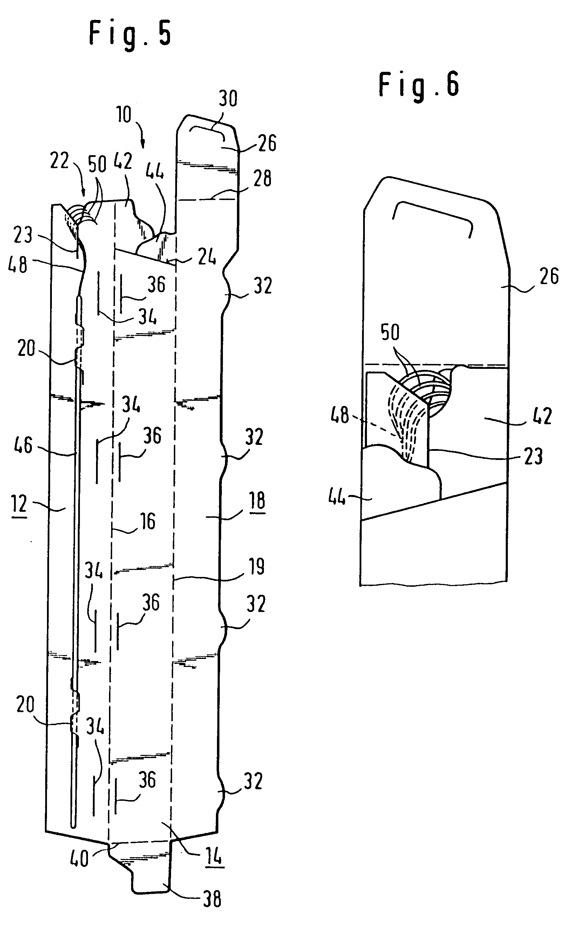

Figs. 5 and 6 show package in which the foam needle park is eliminated and needles 50 and sutures 48 are placed to overlay cutout 22 and slit 23 of panel 12. Tubular member 46 is secured in die-cuts 20 as previously described, and package 10 is folded as described above and as seen in Fig. 6 . To secure needles 50, flap 44 is folded over sutures 48, and then flap 42 is folded over needles 50 as shown. The edge of flap 42 is inserted into slit 23 to hold needles 50 in place. Flap 26 is folded over the assembly as described above. Alternate embodiments of the package of the present invention are of course possible. To engage tabs 32 slots 34 may be sufficient, with slots 36 being omitted. Package 80 of Fig. is identical to package 10 except that the tab and slot arrangement is eliminated and adhesive strip 60 is provided to secure the third panel 18 to first panel 12.

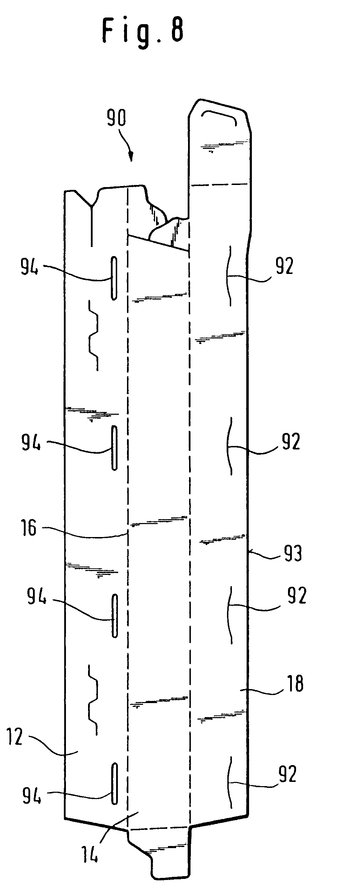

In Figure 8, package 90 is similar to package 10 except for the provision of inwardly directed tabs 92 which are die-cut into panel 18. Panel 18 accordingly has a straight longitudinal edge 93, which overlaps first panel 12, and if desired, may overlay scored line 16 between panels 12 and 14. Tabs 92 engage openings 94 in panel 12 to hold the package 90 in a folded and closed position.

## Claims

1. An elongate package (10) for surgical sutures and suture needle assemblies comprising three interconnected panels, namely, a first panel (12) serving as a suture retaining panel, a second panel (14) connected to said first panel and serving as a front cover, when the panels are folded, and a third panel (18) connected to said second panel and serving as a back cover, with fold lines (16, 19) between the respective panels which extend the length of the package, wherein said first panel includes means (46) for retaining said sutures in an unfolded condition in said package, in order that the suture needles may be located at a first end of the package;
the package being characterised in that:
each of the first and third panels has a needle display flap (42, 44) which extends from the respective lengthwise fold line across the first end of the second panel.

2. A package as claimed in claim 1, wherein said first panel is a needle retaining panel in that it is provided with means for retaining needles of suture needle assemblies.

3. A package as claimed in claims 1 or 2, wherein said second panel includes an extension panel (38) at a second end of said package which folds over to provide a package bottom.

4. A package as claimed in claim 1, 2 or 3, wherein said third panel includes a fold over extension panel (26) at said first end of said package to provide a package top.

5. A package as claimed in any one of the preceding claims, wherein said third panel further includes holding means (32) for holding said package in a fully folded and closed position.

6. A package as claimed in claim 5, wherein the holding means co-operates with said first panel.

7. A package as claimed in claim 6, wherein said holding means comprises at least one tab (32) which interlocks with at least one corresponding slot (34) on said first panel.

8. A package as claimed in claim 7, wherein said at least one tab (32) extends outwardly from a longitudinal edge of said third panel.

9. A package as claimed in claims 7 or 8, wherein said at least one tab (32) comprises an inwardly directed die-cut forming an edge which engages said at least one slot (34) on said first panel.

10. A package as claimed in claims 5 or 6, wherein said holding means comprises an adhesive strip (60) which secures said third panel to said first panel to hold said package in the closed position.

11. A package according to any one of the preceding claims, wherein said means for retaining said sutures comprises an elongate tubular member (46), whereby said sutures can be disposed in said tubular member in an unfolded condition.

12. A package according to claim 11, wherein said first panel includes means (20) for retaining said tubular member (46).

13. A package as claimed in claim 12, wherein said retaining means comprises a die-cut (20).

14. A package as claimed in claim 13, wherein said die-cut (20) is foldable to grasp said tubular member.

15. A package as claimed in claim 12, wherein said means for retaining said suture retaining means (46) comprises adhesives.

16. A package as claimed in claim 2 or any one of claims 3 to 15 as dependent on claim 2, wherein said needle retaining means comprises an adhesive-backed foam needle park (52), said park being positioned so as to overlap an edge of said first panel to adhere to said third panel when said package is in the closed position.

17. A package as claimed in any one of the preceding claims, wherein said needle display flap (42) on said first panel engages a slit (23) in said first panel (12) to hold said needles of said suture needle assemblies.

18. A method of loading sutures into an elongate surgical suture package (10), said package having three interconnected panels (12, 14, 18) joined by longitudinal scored lines, (16, 19) with a pair of needle display flaps (42, 44) attached to each their panel and extending towards each other laterally at one end of the package from one end of the scored lines in an unfolded condition of the package, said method comprising:
placing at least one needled suture in an elongate tubular member (46) with the needle at a first end of the package;
securing said tubular member to a first panel (12) of said package;
folding said first panel having said tubular member containing said at least one suture over a second panel (14) of said package;
folding a third panel (18) over said first panel; and securing said third panel to said first panel to maintain said package in a fully folded and closed condition.

19. A method according to claim 18, comprising the step of folding said needle display flaps (42, 44) over said first panel after said first panel is folded over said second panel.

20. A method according to claim 18 or 19, wherein said third panel includes a fold over extension panel (26), said method further including the step of folding said fold over extension panel over said second and first panel and securing said flap to said second panel after said third panel is folded over said first panel and secured thereto.

21. A method according to claim 18, 19 or 20, wherein said step of securing said tubular member to said first panel comprises positioning said tubular member within a plurality of overlapping die-cut flaps (20) to secure said tubular member.

22. A method according to any one of claims 18 to 21, wherein a plurality of sutures (48) are positioned in said tubular member (46), said sutures having needles (50) attached to one end, said method further comprising the step of securing said needles to said package after said tubular member is secured to said first panel.

23. A method according to any one of claims 18 to 22, wherein said method further comprising folding one (42) of said pair of needle display flaps over said needles and securing said flap member in a slit (23) in said first panel (12) to secure said needles to said package.

24. A method for packaging surgical suture-needle assemblies in an elongate package including three interconnected panels, namely, a first panel (12) serving as a suture and needle retaining panel, a second panel (14) connected to said first panel and serving as a front cover, and a third panel (18) connected to said second panel and serving as a back cover, with fold lines (16, 19) between the respective panels which lines extend the length of the package, said first panel including a tubular member (46) for holding said suture and needle assemblies in an unfolded condition, said package including needle display means (42, 44) at a first end of said package, the second panel including a fold over extension panel (38) at a second end of said package, the needle display means comprising a pair of needle display flaps (42, 44) attached to the first and third panel respectively and extending towards each other laterally from one of said fold lines, said third panel including a fold over extension panel (26) at said first end of said package, and said third panel further including holding means (32) which co-operates with at least said first panel for holding said package in a fully folded and closed position, said method comprising:
placing at least one suture-needle assembly in said tubular member;
securing said tubular member to said first panel;
securing said needles to said first panel;
folding said first panel over said second panel;
folding said second panel fold over extension panel (38) over said first panel;
folding said third panel over said first panel;
securing said third panel to at least said first panel to maintain said package in a closed condition;
folding said third panel fold over extension panel (26) over said first and second panel and securing said extension panel to said second panel to fully close said package.

25. A method according to claim 24, further comprising the step of folding said flap members (42, 44) over said needles and engaging one (42) of said flap members in a slit (23) in said first panel to secure said needles to said first panel.

26. A method according to any one of claims 18 to 25, wherein said steps of securing said needles to said first panel comprises first positioning said needles in a foam needle park and then securing the needle park to said first panel.

27. A method of removing a surgical suture-needle assembly from an elongate package (10), said package including three interconnected panels, namely, a first panel (12) having said suture needle assembly (48) secured thereto in an elongate tubular member (46), said needle assembly being secured in a foam needle park (52) on said first panel, said first panel being folded over a second panel (14), said package having a pair of flap members (42, 44) forming needle display means at a first end and an extension panel (38) folded over said first panel at a second end, and a third panel (18) folded over said first panel and secured thereto to maintain said package in a fully closed position, said third panel including a fold over extension panel (26) at said first end which is folded over said first and second panels and secured to said second panel said pair of flap members being attached to said first (12) and third (18) panels, respectively; said method comprising:
opening said package by unfolding said third panel extension panel (26) to reveal said needle display means (42, 44);
unfolding said flap members (42, 44) of said needle display means to reveal said needle;
grasping said needle (48); and
removing said suture-needle assembly from said package by sliding said assembly out of said elongate tubular member (46);
wherein said suture is maintained in an unfolded condition by said tubular member.

## Patentansprüche

1. Langgestreckte Verpackung (10) für chirugische Nahtmaterialien und Nahtmaterial-Nadelkombinationen umfassend drei miteinander verbundene Platten, nämlich eine erste Platte (12), die als Nahtmaterialhalteplatte dient, eine zweite Platte (14), die mit der ersten Platte verbunden ist und als eine vordere Abdeckung dient, wenn die Platten gefaltet sind, und eine dritte Platte (18), die mit der zweiten Platte verbunden ist und als eine hintere Abdeckung dient, mit Faltungslinien (16, 19) zwischen den jeweiligen Platten, die sich über die Länge der Verpackung erstrecken, wobei die erste Platte eine Einrichtung (46) aufweist, um die Nahtmaterialien in einem nicht gefalteten Zustand in der Verpackung zu halten, damit die Nahtmaterialnadeln an einem ersten Ende der Verpackung angeordnet sein können;
wobei die Verpackung dadurch **gekennzeichnet** ist, daß
jede der ersten und dritten Platten eine Nadelauslageklappe (42, 44) besitzt, die sich von den entsprechenden Faltungslinien in Längsrichtung über das erste Ende der zweiten Platte erstreckt.

2. Verpackung gemäß Anspruch 1, wobei die erste Platte eine Nadelhalteplatte ist, indem sie mit einer Einrichtung zum Halten von Nadeln von Nahtmaterial-Nadelkombinationen versehen ist.

3. Verpackung gemäß Anspruch 1 oder 2, wobei die zweite Platte eine Ansatzplatte (38) an einem zweiten Ende der Verpackung aufweist, die sich umfaltet, um einen Verpackungsboden bereitzustellen.

4. Verpackung gemäß Anspruch 1, 2, oder 3, wobei die dritte Platte eine Überfalt-Ansatzplatte (26) an dem ersten Ende der Verpackung aufweist, um eine Verpackungsoberseite bereitzustellen.

5. Verpackung gemäß einem der vorhergehenden Ansprüche, wobei die dritte Platte weiter eine Halteeinrichtung (32) aufweist, um die Verpackung in einer vollständig gefalteten und geschlossenen Position zu halten.

6. Verpackung gemäß Anspruch 5, wobei die Halteeinrichtung mit der ersten Platte zusammenwirkt.

7. Verpackung gemäß Anspruch 6, wobei die Halteeinrichtung zumindest eine Nase (32) umfaßt, die sich mit zumindest einem entsprechenden Schlitz (34) auf der ersten Platte verriegelt.

8. Verpackung gemäß Anspruch 7, wobei die zumindest eine Nase (32) sich von einer Längskante der dritten Platte nach außen erstreckt.

9. Verpackung gemäß Anspruch 7 oder 8, wobei die zumindest eine Nase (32) eine nach innen gerichtete Ausstanzung umfaßt, welche eine Kante bildet, die in den zumindest einen Schlitz (34) auf der ersten Platte einrückt.

10. Verpackung gemäß Anspruch 5 oder 6, wobei die Halteeinrichtung einen Klebstoffstreifen (60) umfaßt, der die dritte Platte an der ersten Platte befestigt, um die Verpackung in der geschlossenen Position zu halten.

11. Verpackung gemäß einem der vorhergehenden Ansprüche, wobei die Einrichtung zum Halten der Nahtmaterialien ein langgestrecktes röhrenförmiges Element (46) umfaßt, wobei die Nahtmaterialien in dem röhrenförmigen Element in einem nicht gefalteten Zustand angeordnet werden können.

12. Verpackung gemäß Anspruch 11, wobei die erste Platte eine Einrichtung (20) zum Halten des röhrenförmigen Elements (46) umfaßt.

13. Verpackung gemäß Anspruch 12, wobei die Halteeinrichtung einen ausgestanzten Schnitt (20) umfaßt.

14. Verpackung gemäß Anspruch 13, wobei der ausgestanzte Schnitt (20) faltbar ist, um das röhrenförmige Element zu greifen.

15. Verpackung gemäß Anspruch 12, wobei die Einrichtung zum Halten der Nahtmaterialhalteeinrichtung (46) Klebstoffe umfaßt.

16. Verpackung gemäß Anspruch 2 oder einem der Ansprüche 3 bis 15, sofern diese von Anspruch 2 abhängig sind, wobei die Nadelhalteeinrichtung eine Schaumnadelauflage (52) mit Kleberückseite umfaßt, wobei die Auflage so angeordnet ist, daß sie eine Kante der ersten Platte überlappt, um an der dritten Platte anzuhaften, wenn die Verpackung in der geschlossenen Position ist.

17. Verpackung gemäß einem der vorhergehenden Ansprüche, wobei die Nadelauslageklappe (42) auf der ersten Platte in einen Schlitz (23) in der ersten Platte (12) einrückt, um die Nadeln der Nahtmaterial-Nadelkombinationen zu halten.

18. Verfahren zum Einsetzen von Nahtmaterialien in eine langgestreckte chirugische Nahtmaterialverpackung (10), wobei die Verpackung drei miteinander verbundene Platten (12, 14, 18) besitzt, die durch längsverlaufende Faltungslinien (16, 19) verbunden sind mit einem Paar von Nadelauslageklappen (42, 44), die an jeweils ihrer Platte angebracht sind und sich seitlich aufeinander zu an einem Ende der Verpackung von einem Ende der Faltungslinien in einem nicht gefalteten Zustand der Verpackung erstrecken, wobei das Verfahren umfaßt:
• Einsetzen zumindest eines mit Nadel versehenen Nahtmaterials in ein langgestrecktes röhrenförmiges Element (46) mit der Nadel an einem ersten Ende der Verpackung;
• Befestigen des röhrenförmigen Elementes an einer ersten Platte (12) der Verpackung;
• Falten der ersten Platte mit dem röhrenförmigen Element, das zumindest ein Nahtmaterial enthält, über eine zweite Platte (14) der Verpackung;
• Falten einer dritten Platte (18) über die erste Platte; und
• Befestigen der dritten Platte an der ersten Platte, um die Verpackung in einem vollständig gefalteten und geschlossenen Zustand zu halten.

19. Verfahren gemäß Anspruch 18, umfassend den Schritt des Faltens der Nadelauslageklappen (42, 44) über die erste Platte, nachdem die erste Platte über die zweite Platte gefaltet worden ist.

20. Verfahren gemäß Anspruch 18 oder 19, wobei die dritte Platte eine Ansatzplatte (26) zum Umfalten aufweist, und das Verfahren weiter umfaßt den Schritt des Faltens der umfaltbaren Ansatzplatte über die zweite und erste Platte und des Befestigens der Klappe an der zweiten Platte, nachdem die dritte Platte über die erste Platte gefaltet worden und an dieser befestigt worden ist.

21. Verfahren gemäß Anspruch 18, 19 oder 20, wobei der Schritt des Befestigens des röhrenförmigen Elementes an der ersten Platte umfaßt das Anordnen des röhrenförmigen Elementes innerhalb einer Mehrzahl von überlappenden stanzgeschnittenen Klappen (20), um das röhrenförmige Element zu befestigen.

22. Verfahren gemäß einem der Ansprüche 18 bis 21, wobei eine Mehrzahl von Nahtmaterialien (48) in dem röhrenförmigen Element (46) angeordnet sind, und die Nahtmaterialien Nadeln (50) an einem Ende angebracht besitzen, wobei das Verfahren weiter umfaßt den Schritt des Befestigens der Nadeln an der Verpackung, nachdem das röhrenförmige Element an der ersten Platte befestigt worden ist.

23. Verfahren gemäß einem der Ansprüche 18 bis 22, wobei das Verfahren weiter umfaßt des Faltens einer (42) des Paares von Nadelauslageklappen über die Nadeln und das Befestigen des Klappenelementes in einem Schlitz (23) in der ersten Platte (12), um die Nadeln an der Verpackung zu befestigen.

24. Verfahren zum Verpacken chirugischer Nahtmaterial-Nadelkombinationen in einer langgestreckten Verpackung umfassend drei miteinander verbundene Platten, nämlich eine erste Platte (12), die als eine Nahtmaterial- und Nadelhalteplatte dient, eine zweite Platte (14), die mit der ersten Platte verbunden ist und als eine vordere Abdeckung dient, und eine dritte Platte (18), die mit der zweiten Platte verbunden ist und als eine hintere Abdeckung dient, mit Faltungslinien (16, 19) zwischen den entsprechenden Platten, die sich über die Länge der Verpackung erstrecken, wobei die erste Platte ein röhrenförmiges Element (46) zum Halten der Nahtmaterial- und Nadelkombinationen in einem nicht gefalteten Zustand aufweist, die Verpackung eine Nadelauslageeinrichtung (42, 44) am ersten Ende der Verpackung aufweist, die zweite Platte eine umfaltbare Ansatzplatte (38) an einem zweiten Ende der Verpackung aufweist, die Nadelauslageeinrichtung ein Paar von Nadelauslageklappen (42, 44) umfaßt, die an der ersten und dritten Platte jeweils angebracht sind und sich in Richtung aufeinander seitlich zu von einer der Faltungslinien erstrecken, wobei die dritte Platte eine umfaltbare Ansatzplatte (26) an dem ersten Ende der Verpackung aufweist und die dritte Platte weiter eine Halteeinrichtung (32) aufweist, die mit zumindest der ersten Platte zusammenwirkt, um die Verpackung in der vollständig gefalteten und geschlossenen Position zu halten, wobei das Verfahren umfaßt:
• Einsetzen zumindest einer Nahtmaterial-Nadelkombination in das röhrenförmige Element;
• Befestigen des röhrenförmigen Elementes an der ersten Platte;
• Befestigen der Nadeln an der ersten Platte;
• Falten der ersten Platte über die zweite Platte;
• Falten der zweiten Platte über die Ansatzplatte (38) über die erste Platte;
• Falten der dritten Platte über die erste Platte;
• Befestigen der dritten Platte an zumindest der ersten Platte, um die Verpackung in einem geschlossenen Zustand zu halten;
• Falten der faltbaren Ansatzplatte (26) der dritten Platte über die erste und zweite Platte und Befestigen der Ansatzplatte an der zweiten Platte, um die Verpackung vollständig zu schließen.

25. Verfahren gemäß Anspruch 24 weiter umfassend den Schritt des Faltens der Klappenelemente (42, 44) über die Nadeln und des Einrückens eines (42) der Klappenelemente in einen Schlitz (23) in der ersten Platte, um die Nadeln an der ersten Platte zu befestigen.

26. Verfahren gemäß einem der Ansprüche 18 bis 25, wobei die Schritte des Befestigens der Nadeln an der ersten Platte umfassen zuerst das Anordnen der Nadeln in einer Schaumnadelauflage und dann das Befestigen der Nadelauflage an der ersten Platte.

27. Verfahren zum Entfernen einer chirugischen Nahtmaterial-Nadelkombination von einer langgestreckten Verpackung (10), wobei die Verpackung drei miteinander verbundene Platten umfaßt, nämlich eine erste Platte (12) mit der daran in einem langgestreckten röhrenförmigen Element (46) befestigten Nahtmaterial-Nadelkombination (48), wobei die Nadelkombination in einer Schaumnadelauflage (52) auf der ersten Platte befestigt ist, die erste Platte über eine zweite Platte (14) umgefaltet ist, die Verpackung ein Paar von Klappenelementen (42, 44) besitzt, die eine Nadelauslageeinrichtung an einem ersten Ende bilden und eine Erweiterungsplatte (38), die über die erste Platte an einem zweiten Ende gefaltet ist, und eine dritte Platte (18), die über die erste Platte gefaltet ist und an dieser befestigt ist, um die Verpackung in einer vollständig geschlossenen Position zu halten, wobei die dritte Platte eine umfaltbare Ansatzplatte (26) an dem ersten Ende aufweist, die über die ersten und zweiten Platten umgefaltet und an der zweiten Platte befestigt ist, wobei das Paar von Klappenelementen an den ersten (12) und dritten (18) Platten jeweils angebracht ist, und das Verfahren umfaßt:
• Öffnen der Verpackung durch das Entfalten der Erweiterungsplatte (26) der dritten Platte, um die Nadelauslageeinrichtung (42, 44) freizulegen;
• Entfalten der Klappenlemente (42, 44) der Nadelauslageeinrichtung, um die Nadel freizulegen;
• Greifen der Nadel (48); und
• Entfernen der Nahtmaterial-Nadelkombination von der Verpackung, indem die Kombination aus dem langgestreckten röhrenförmigen Element (46) herausgezogen wird;
• wobei das Nahtmaterial durch das röhrenförmige Element in einem nicht gefalteten Zustand gehalten wird.

## Revendications

1. Emballage de forme oblongue (10) pour des ensembles de suture et de suture-aiguille chirurgicaux comprenant trois panneaux interconnectés, à savoir un premier panneau (12) servant de panneau retenant le matériau de suture, un deuxième panneau (14) relié audit premier panneau et servant de recouvrement avant, lorsque les panneaux sont pliés, et un troisième panneau (18) relié audit deuxième panneau et servant de recouvrement arrière, avec des lignes de pliage (16, 19) entre les panneaux respectifs qui s'étendent sur la longueur de l'emballage, où ledit premier panneau comprend des moyens (46) pour retenir lesdites sutures dans un état déplié dans ledit emballage pour que les aiguilles de suture puissent être situées à une première extrémité de l'emballage;
l'emballage étant caractérisé en ce que :
chacun des premier et troisième panneaux a un volet de présentation (42, 44) des aiguilles qui s'étend de la ligne de pliage longitudinale respective sur la première extrémité du deuxième panneau.

2. Emballage selon la revendication 1, où ledit premier panneau est un panneau de retenue des aiguilles en ce qu'il est pourvu de moyens pour retenir les aiguilles des ensembles de suture-aiguille.

3. Emballage selon les revendications 1 ou 2, où ledit deuxième panneau comporte un panneau d'extension (38) à une deuxième extrémité dudit emballage qui est replié pour constituer un fone d'emballage.

4. Emballage selon la revendication 1, 2 ou 3, où ledit troisième panneau comporte un panneau d'extension de repliage (26) à ladite première extrémité dudit emballage pour constituer un dessus d'emballage.

5. Emballage selon l'une des revendications précédentes, où ledit troisième panneau comporte en outre des moyens de retenue (32) pour tenir l'emballage dans une position entièrement pliée et fermée.

6. Emballage selon la revendication 5, où les moyens de retenue coopèrent avec ledit premier panneau.

7. Emballage selon la revendication 6, où ledit moyen de retenue comporte au moins une patte (32) qui est interverrouillée avec au moins une fente correspondante (34) sur ledit premier panneau.

8. Emballage selon la revendication 7, où ladite au moins une patte (32) s'étend vers l'extérieur depuis un bord longitudinal dudit troisième panneau.

9. Emballage selon les revendications 7 ou 8, où ladite au moins une patte (32) comprend une découpure de matrice dirigée vers l'intérieur formant un bord qui s'engage dans ladite au moins une fente (34) sur ledit premier panneau.

10. Emballage selon les revendications 5 ou 6, où ledit moyen de retenue comprend une bande adhésive (60) qui fixe ledit troisième panneau audit premier panneau pour maintenir l'emballage dans la position fermée.

11. Emballage selon l'une des revendications précédentes, où ledit moyen pour retenir lesdites sutures comprend un élément tubulaire oblong (46) par quoi lesdites sutures peuvent être disposées dans ledit élément tubulaire dans un état déplié.

12. Emballage selon la revendication 11, où ledit premier panneau comprend un moyen (20) pour retenir ledit élément tubulaire (46).

13. Emballage selon la revendication 12, où ledit moyen de retenue comprend une découpure de matrice (20).

14. Emballage selon la revendication 13, où ladite découpure de matrice (20) est pliable pour saisir ledit élément tubulaire.

15. Emballage selon la revendication 12, où ledit moyen pour retenir ledit moyen de retenue de suture (46) comprend des adhésifs.

16. Emballage selon la revendication 2 ou l'une des revendications 3 à 15 telles que dépendantes de la revendication 2, où ledit moyen de retenue d'aiguille comprend un emplacement d'aiguilles en mousse à support adhésif (52), ledit emplacement étant positionné de façon à recouvrir un bord dudit premier panneau pour adhérer audit troisième panneau lorsque ledit emballage se trouve dans la position fermée.

17. Emballage selon l'une des revendications précédentes, où ledit volet de présentation (42) des aiguilles sur ledit premier panneau s'engage dans une fente (23) dans ledit premier panneau (12) pour retenir lesdites aiguilles desdits ensembles à fils de suture et à aiguilles.

18. Procédé pour charger des matériaux de suture dans un emballage de suture chirurgical oblong (10), ledit emballage ayant trois panneaux interconnectés (12, 14, 18) reliés par des lignes entaillées longitudinales (16, 19), une paire de volets de présentation des aiguilles (42, 44) étant attachée à chacun de leurs panneaux et s'étendant l'un vers l'autre latéralement à une extrémité de l'emballage d'une extrémité des lignes entaillées, dans un état déplié de l'emballage, ledit procédé comprenant les étapes consistant à :
placer au moins un fil de suture à aiguille dans un élément tubulaire oblong (46), l'aiguille se trouvant à une première extrémité de l'emballage;
fixer ledit élément tubulaire à un premier panneau (12) dudit emballage;
plier ledit premier panneau ayant ledit élément tubulaire contenant ladite au moins une suture sur un deuxième panneau (14) dudit emballage;
plier un troisième panneau (18) sur ledit premier panneau; et fixer ledit troisième panneau audit premier panneau pour maintenir ledit emballage dans un état entièrement plié et fermé.

19. Procédé selon la revendication 18, comprenant l'étape consistant à plier lesdits volets de présentaion (42, 44) des aiguilles sur ledit premier panneau après avoir plié ledit premier panneau sur ledit deuxième panneau.

20. Procédé selon la revendication 18 ou 19, où ledit troisième panneau comporte un panneau d'extension de repliage (26), ledit procédé incluant en outre l'étape consistant à plier ledit panneau d'extension de repliage sur lesdits deuxième et premier panneaux et de fixer ledit volet audit deuxième panneau après avoir plié ledit troisième panneau sur ledit premier panneau et l'avoir fixé à celui-ci.

21. Procédé selon la revendication 18, 19 ou 20, où ladite étape de fixation dudit élément tubulaire audit premier panneau comprend le positionnement dudit élément tubulaire dans une pluralité de volets chevauchés découpés à la matrice (20) pour immobiliser ledit élément tubulaire.

22. Procédé selon l'une des revendications 18 à 21, où une pluralité de fils de suture (48) sont positionnés dans ledit élément tubulaire (46), lesdits fils de suture ayant des aiguilles (50) attachées à une extrémité, ledit procédé comprenant en outre l'étape consistant à fixer lesdites aiguilles audit emballage après que ledit élément tubulaire soit fixé audit premier panneau.

23. Procédé selon l'une des revendications 18 à 22, où ledit procédé comprend en outre le pliage d'un (42) de ladite paire de volets de présentation d'aiguille sur lesdites aiguilles et la fixation dudit élément de volet dans une fente (23) dans ledit premier panneau (12) pour fixer lesdites aiguilles audit emballage.

24. Procédé pour emballer des ensembles de suture-aiguille chirurgicaux dans un emballage de forme oblongue incluant trois panneaux interconnectés, à savoir un premier panneau (12) servant de panneau de retenue de fil de suture et d'aiguille, un deuxième panneau (14) relié audit premier panneau et servant de recouvrement avant et un troisième panneau (18) relié audit deuxième panneau et servant de recouvrement arrière, avec des lignes de pliage (16, 19) entre les panneaux respectifs, lesdites lignes s'étendant sur la longueur de l'emballage, ledit premier panneau incluant un élément tubulaire (46) pour tenir lesdits ensembles de suture et d'aiguille dans un état déplié, ledit amballage incluant des moyens de présentation d'aiguille (42, 44) à une première extrémité dudit emballage, le deuxième panneau incluant un panneau d'extension de repliage (38) à une deuxième extrémité dudit amballage, le moyen de présentation d'aiguille comprenant une paire de volets de présentation d'aiguille (42, 44) attachées au premier et troisième panneaux, respectivement et s'étandant l'un vers l'autre latéralement depuis l'une desdites lignes de pliage ledit troisième panneau incluant un panneau d'extension de repliage (26) à ladite première extrémité dudit emballage, et ledit troisième panneau incluant en outre un moyen de retenue (32) qui coopère avec au moins ledit premier panneau pour tenir ledit emballage dans une position entièrement pliée et fermée, ledit procédé comprenant les étapes consistant à :
placer au moins un ensemble à fils de suture et à aiguilles dans ledit élément tubulaire;
fixer ledit élément tubulaire audit premier panneau;
fixer lesdites aiguilles audit premier panneau;
plier ledit premier panneau sur ledit deuxième panneau;
plier ledit panneau d'extension de repliage (38) du deuxième panneau sur ledit premier panneau;
plier ledit troisième panneau sur ledit premier panneau;
fixer ledit troisième panneau à au moins ledit premier panneau pour maintenir ledit emballage dans un état fermé;
plier ledit panneau d'extension de repliage (26) du troisième panneau sur lesdits premier et deuxième panneaux et fixer ledit panneau d'extension audit deuxième panneau pour fermer entièrement ledit emballage.

25. Procédé selon la revendication 24, comprenant en outre l'étape consistant à plier lesdits éléments de volet (42, 44) sur lesdites aiguilles et à engager l'un (42) desdits éléments de volet dans une fente (23) dans ledit premier panneau pour fixer lesdites aiguilles audit premier panneau.

26. Procédé selon l'une des revendications 18 à 25, où ladite étape consistant à fixer lesdites aiguilles audit premier panneau comprend d'abord le positionnement desdites aiguilles dans un emplacement d'aiguilles en mousse et ensuite la fixation de l'emplacement d'aiguilles audit premier panneau.

27. Procédé pour retirer un ensemble de suture-aiguille chirurgical d'un emballage de forme oblongue (10), ledit emballage incluant trois panneaux interconnectés, à savoir un premier panneau (12) ayant ledit ensemble de suture-aiguille (48) fixé à celui-ci dans un élément tubulaire allongé (46), ledit ensemble d'aiguilles étant fixé dans un emplacement d'aiguilles en mousse (52) sur ledit premier panneau, ledit premier panneau étant plié sur un deuxième panneau (14), ledit emballage ayant une paire d'éléments de volet (42, 44) formant un moyen de présentation des aiguilles à une première extrémité et un panneau d'extension (38) plié sur ledit premier panneau à une deuxième extrémité, et un troisième panneau (18) plié sur ledit premier panneau et fixé à celui-ci afin de maintenir ledit emballage dans une position entièrement fermée ledit troisième panneau incluant un panneau d'extension de repliage (26) à ladite première extrémité qui est plié sur lesdits premier et deuxième panneaux et fixé audit deuxième panneau, ladite paire d'éléments de volet étant fixée audit premier (12) et troisième (18) panneaux, respectivement; ledit procédé comprenant les étapes consistant à :
ouvrir ledit emballage en dépliant le panneau d'extension du troisième panneau (26) pour découvrir ledit moyen de présentation des aiguilles (42, 44);
déplier lesdits éléments de volet (42, 44) dudit moyen de présentation des aiguilles pour présenter ladite aiguille;
saisir ladite aiguille (48); et
retirer ledit ensemble de suture-aiguille dudit emballage en faisant glisser ledit ensemble hors dudit élément tubulaire allongé (46);
où ledit matériau de suture est maintenu dans un état déplié par ledit élément tubulaire.
